# EUROPEAN PATENT APPLICATION

(11) **EP 1 983 339 A1**
(43) Date of publication of application: **22.10.2008**
(21) Application number: 07707132.2
(22) Date of filing: 21.01.2007
(51) Int. Cl.: G01N 27/28, G01N 27/327, G01N 27/416

(54) **ANALYSIS DEVICE HAVING A DISCARDING MECHANISM**

(30) Priority: 22.01.2006 JP 2006013260
(71) Applicant: Arkray, Inc., Kyoto-shi, Kyoto 601-8045 (JP)
(72) Inventor: SAKATA, Tetsuya, Kyoto-shi, Kyoto, 601-8045 (JP); MURAKAMI, Shinsui, Kyoto-shi, Kyoto, 601-8045 (JP)
(74) Representative: Wiedemann, Peter
(86) International application number: PCT/JP2007/050844
(87) International publication number: WO 2007/083773

(57) **Abstract**

An analyzing device which fits an analyzing instrument 2 thereinto for use and has a disposal mechanism 5 for disposing of the analyzing instrument 2 after completion of analysis, wherein the disposal mechanism 5 inputs a load in a direction different from a disposal direction D1 of the analyzing instrument 2 to dispose of the analyzing instrument 2. In the analyzing device 1 having a connector portion 4 having a terminal to be brought into contact with a terminal of the analyzing instrument 2, the disposal mechanism 4 is arranged below the connector portion 4.

## Description

### TECHNICAL FIELD

The present invention relates to an analyzing device which fits an analyzing instrument thereinto for use and has a disposal mechanism for disposing of the analyzing instrument after completion of analysis.

### BACKGROUND ART

In a method for measuring a blood sugar level, an analyzing instrument has hitherto been used. As an example, the user fits the analyzing instrument into a portable blood sugar level measuring device to spot blood onto the analyzing instrument, and then, the blood sugar level measuring device automatically measures a blood sugar level.

In the principle for measuring a blood sugar level, for example, an electrochemical method or an optical method is used. When the electrochemical method is used for blood sugar level measurement, the analyzing instrument has an electrode for applying a voltage. The analyzing device measuring device applies a voltage to the electrode of the analyzing instrument and can measure its responding current. On the other hand, when the optical method is used for blood sugar level measurement, the analyzing instrument has a reagent portion which exhibits coloration according to the concentration of a blood sugar level. The blood sugar level measuring device has a photometer mechanism for photometry of the degree of coloration of the reagent portion.

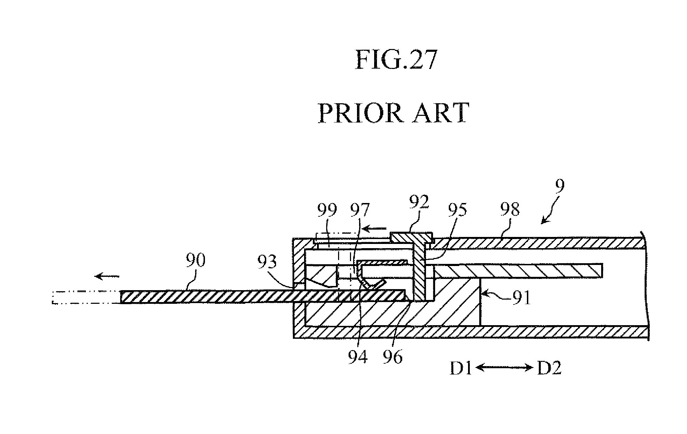

Meanwhile, there has been proposed the blood sugar level measuring device which has a mechanism for disposing of the analyzing instrument as illustrated in Fig. 27 so as to detach the analyzing instrument fitted into the blood sugar level measuring device without touching it (for example, refer to Patent Documents 1 and 2).

A blood sugar level measuring device 9 illustrated in the drawing analyzes a specimen using the electrochemical method with an analyzing instrument 90, and has a connector portion 91 and an operating knob 92. The connector portion 91 fits the analyzing instrument 90 thereinto, applies a voltage to an electrode (not illustrated) of the analyzing instrument 90, and measures its responding current. The operating knob 92 is used for disposing of the analyzing instrument 90 fitted into the connector portion 91 and can be slid in D1 and D2 directions.

The analyzing instrument 90 is fitted into the blood sugar level measuring device 9 through an insertion opening 93, so that a terminal 94 of the connector portion 91 is brought into contact with the electrode (not illustrated) of the analyzing instrument 90. In this state, the connector portion 91 can apply a voltage to the electrode of the analyzing instrument 90 and measure a responding current. The blood sugar level measuring device 9 can measure the glucose concentration (blood sugar level) of blood supplied to the analyzing instrument 90 based on the responding current.

When the blood sugar level measurement is completed, the operating knob 92 is moved in the D1 direction to dispose of the analyzing instrument 90 from the blood sugar level measuring device 9. That is, the operating knob 92 is moved in the D1 direction to press an interfering portion 95 of the operating knob 92 onto an end face 96 of the analyzing instrument 90. Accordingly, movement of the operating knob 92 in the D1 direction pushes out the analyzing instrument 90 in the D1 direction.

However, in the blood sugar level measuring device 9, the end face 96 of the analyzing instrument 90 is pressed by the interfering portion 95 of the operating knob 92. Thus, the connector portion 91 and a housing 98 need to allow movement of the interfering portion 95.

Therefore, a slit 97 for securing the moving path of the interfering portion 95 need to be provided in the connector portion 91. As a result, the connector portion 91 in the blood sugar level measuring device not having the disposal mechanism cannot be directly diverted without changing the design and need to change the design. When the slit 97 is provided in the connector portion 91, the connector portion 91 is increased in size. Furthermore, when the slit 97 is provided, the component of the connector portion 91 is susceptible to layout limit. In this point, there is the problem with design, resulting in increase in the size of the connector portion 91.

When the configuration which disposes of the analyzing instrument by the operating knob is employed in the blood sugar level measuring device using the optical method, a slit 99 for allowing movement of the interfering portion 95 need to be provided in the housing 98. Therefore, an external light can be easily incident into the analyzing device through the slit 98 of the housing 98. Thus, the photometry result of the photometer mechanism is likely to be affected by the external light. To prevent such disadvantage, means for limiting incidence of the external light through the slit 98 need to be provided. This results in not only a complicated configuration of the analyzing device but an increase in size and cost of the analyzing device.

Patent Document 1: Japanese Patent Application Laid-Open (JP-A) No. 2003-114213
Patent Document 2: Japanese Patent Application Laid-Open (JP-A) No. 2001-33418

### DISCLOSURE OF THE INVENTION

### PROBLEMS TO BE SOLVED BY THE INVENTION

An object of the present invention is to prevent an increase in the size and manufacturing cost of an analyzing device due to provision of a disposal mechanism and to appropriately analyze a specimen.

### MEANS FOR SOLVING THE PROBLEMS

An analyzing device provided according to a first aspect of the present invention which fits an analyzing instrument thereinto for use and has a disposal mechanism for disposing of the analyzing instrument after completion of analysis, wherein the disposal mechanism inputs a load in a direction different from a disposal direction of the analyzing instrument to dispose of the analyzing instrument.

An analyzing device provided according to a second aspect of the present invention includes: a connector portion which fits thereinto a planar analyzing instrument which can analyze a specimen by an electrochemical method and has a terminal brought into contact with a terminal of the analyzing instrument; and a disposal mechanism for disposing of the analyzing instrument after completion of analysis, wherein the disposal mechanism is arranged below the connector portion. In this case, the disposal mechanism preferably inputs a load in a direction different from a disposal direction of the analyzing instrument to dispose of the analyzing instrument.

The disposal mechanism has a moving body which has a contacting portion brought into close contact with the analyzing instrument and can be reciprocated between a standby position and a disposal position which can dispose of the analyzing instrument. In this case, when a load is inputted by the contacting portion and the moving body is moved from the standby position to the disposal position, the analyzing instrument is moved together with the moving body.

The disposal mechanism moves the analyzing instrument close-contacted with the contacting portion due to frictional resistance between the analyzing instrument and the contacting portion by movement of the moving body. In this case, the contacting portion is formed of an elastic member.

When the moving body is in the standby position, the contacting portion is not brought into contact with the analyzing instrument, and when the moving body is moved from the standby position to the disposal position, the contacting portion is brought into contact with the analyzing instrument. In this case, the contacting portion is shifted upward while the moving body is moved from the standby position to the disposal position.

The analyzing device of the present invention further has an engaging portion which engages the moving body and can be moved relatively to the moving body. In this case, one of the moving body and the engaging portion has, for example, a guide groove and the other has a convex portion which engages the guide groove. When the moving body is reciprocated relatively to a housing, the convex portion is moved in the guide groove to shift the contacting portion upward and downward.

The analyzing device of the present invention further has a pressing portion which sandwiches the analyzing instrument between the pressing portion and the contacting portion when the contacting portion is, for example, shifted upward.

The analyzing device of the present invention further has: an operating body which can be reciprocated relatively to the housing; and a link member for converting the reciprocating motion of the operating body to the reciprocating motion of the moving body.

The operating body and the moving body each have a rack portion having a plurality of teeth. The link member is a gear which engages the plurality of teeth of the rack portion of the operating body or the moving body. When the operating body is moved, the moving body is moved in a direction opposite the operating body.

The gear is preferably rotated in a horizontal direction.

The operating body is preferably urged in a direction from the standby position to the disposal position. The analyzing device further has a stopper portion for locating the operating body in the standby position.

The disposal mechanism may input a load to the side surface of the analyzing instrument to dispose of the analyzing instrument. The disposal mechanism has a rotor which has a contacting portion brought into close contact with the analyzing instrument and is rotatable. The analyzing instrument is moved by inputting a load by the contacting portion and rotating the rotor.

The disposal mechanism further has an operating body operated to rotate the rotor. The disposal mechanism further has a link member for transmitting a load inputted to the operating body as the rotational force of the rotor.

The analyzing instrument which has an engaging portion for engaging the contacting portion can be used.

An analyzing device provided according to a third aspect of the present invention which fits an analyzing instrument thereinto for use and has a disposal mechanism which disposes of the analyzing instrument after completion of analysis, wherein the disposal mechanism has a moving body which has a contacting portion brought into close contact with the analyzing instrument and can be reciprocated between a standby position and a disposal position which can dispose of the analyzing instrument, wherein when a load is inputted by the contacting portion and the moving body is moved from the standby position to the disposal position, the analyzing instrument is moved together with the moving body.

### BRIEF DESCRIPTION OF THE DRAWINGS

Fig. 1 is an overall perspective view illustrating an analyzing device according to a first embodiment of the present invention.
Fig. 2 is an exploded perspective view of a portion of the analyzing device illustrated in Fig. 1.
Fig. 3 is a cross-sectional view illustrating the essential portion of the analyzing device illustrated in Fig. 1.
Fig. 4 is a cross-sectional view illustrating the essential portion of the analyzing device illustrated in Fig. 1.
Fig. 5 is an exploded perspective view of a disposal mechanism in the analyzing device illustrated in Fig. 1.
Fig. 6 is a plan view illustrating a moving body in the disposal mechanism illustrated in Fig. 5.
Fig. 7 is a cross-sectional view illustrating the essential portion of a fixing body in the disposal mechanism illustrated in Fig. 5.
Fig. 8 is an overall perspective view illustrating an example of a biosensor used for the analyzing device illustrated in Fig. 1.
Fig. 9 is a cross-sectional view taken along line IX-IX of Fig. 8.
Fig. 10 is an exploded perspective view of the biosensor illustrated in Fig. 8.
Fig. 11 is a cross-sectional view corresponding to Fig. 3 of assistance in explaining the operation of the analyzing device illustrated in Fig. 1.
Fig. 12 is a cross-sectional view corresponding to Fig. 4 of assistance in explaining the operation of the analyzing device illustrated in Fig. 1.
Fig. 13 is a cross-sectional view corresponding to Fig. 7 of assistance in explaining the operation of the analyzing device illustrated in Fig. 1.
Fig. 14 is an essential portion cross-sectional view of assistance in explaining the operation of the analyzing device illustrated in Fig. 1.
Fig. 15 is a cross-sectional view corresponding to Fig. 7 of assistance in explaining the operation of the analyzing device illustrated in Fig. 1.
Fig. 16 is an essential portion cross-sectional view of assistance in explaining the operation of the analyzing device illustrated in Fig. 1.
Fig. 17 is a cross-sectional view corresponding to Fig. 7 of assistance in explaining the operation of the analyzing device illustrated in Fig. 1.
Fig. 18 is an essential portion cross-sectional view of assistance in explaining the operation of the analyzing device illustrated in Fig. 1.
Fig. 19 is an overall perspective view illustrating an analyzing device according to a second embodiment of the present invention.
Fig. 20 is a perspective view illustrating the essential portion of the analyzing device illustrated in Fig. 19.
Fig. 21 is a plan view illustrating the essential portion of the analyzing device illustrated in Fig. 19.
Fig. 22 is a cross-sectional view taken along line XXII-XXII of Fig. 21.
Fig. 23 is a cross-sectional view taken along line XXIII-XXIII of Fig. 21.
Fig. 24 is a plan view illustrating the essential portion of assistance in explaining the operation of the analyzing device illustrated in Fig. 19.
Fig. 25 is a plan view illustrating an example which applies the analyzing device illustrated in Fig. 19 to an analyzing instrument of another embodiment.
Fig. 26 is a cross-sectional view illustrating the essential portion of an analyzing device according to a third embodiment of the present invention.
Fig. 27 is an essential portion cross-sectional view of assistance in explaining a disposal mechanism in an analyzing device in prior art.

### EXPLANATION OF REFERENCE NUMERALS

- 1, 8, 8': Blood sugar level measuring device (analyzing device)
- 2, 2', 2": Biosensor (analyzing instrument)
- 2A: Back surface (of the biosensor) (one side of the analyzing instrument)
- 2B': Concave portion
- 24A: End portion (of an operating electrode) (terminal of the analyzing instrument)
- 25A: End portion (of a counter electrode) (terminal of the analyzing instrument)
- 27: Concave portion (engaging portion) 3 Housing 36 Stopper portion 38 Flange portion (of the housing) (pressing portion) 4 Connector portion 40 Terminal (of the connector portion) 50, 85 Operating body 52 Rack portion (of the operating body) 54 Teeth (of the rack portion of the operating body) 60 Moving body 62 Rack portion (of the moving body)
- 66: Teeth (of the rack portion of the moving body)
- 64: Engaging pin (of the moving body)
- 71: Gear (link member)
- 72: Guide portion (engaging portion)
- 76: Guide groove (of the guide portion)
- 82: Disposal mechanism
- 87: Link arm (of the disposal mechanism) (link member)
- 88: Rotor (of the disposal mechanism)
- 88C: Contacting portion (of the rotor)
- 80': Disposal mechanism
- 81': Moving body (of the disposal mechanism)
- 83': Contacting portion (of the disposal mechanism)

### BEST MODE FOR CARRYING OUT THE INVENTION

First to third embodiments according to the present invention will be described below by taking a blood sugar level measuring device as an example with reference to the drawings.

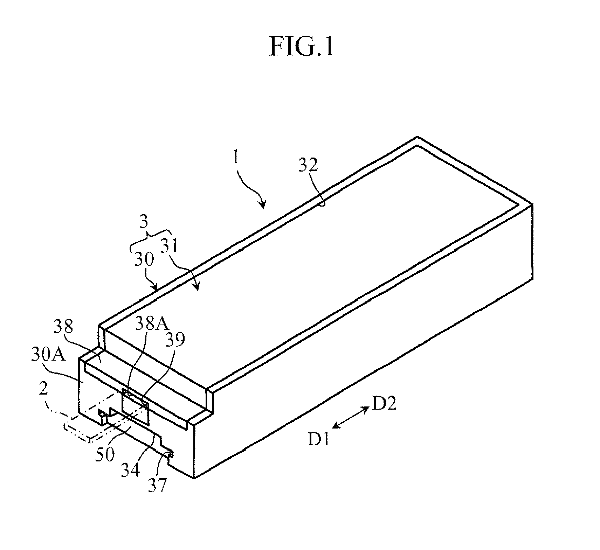

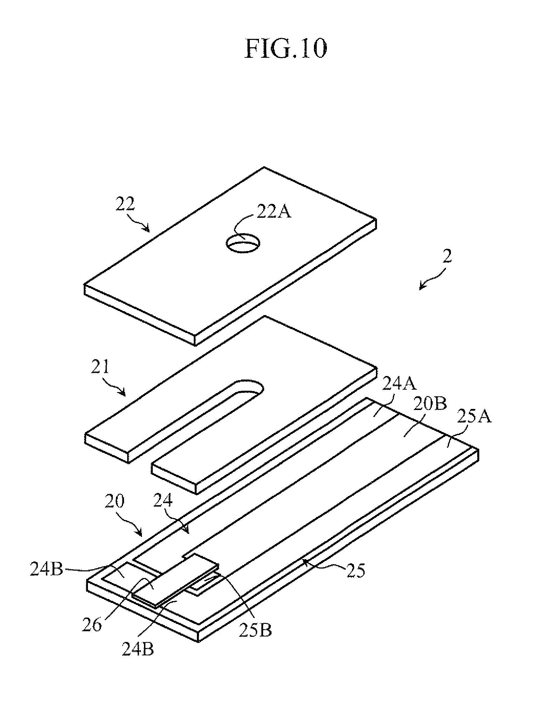

The first embodiment of the present invention will be described with reference to Figs. 1 to 18.

A blood sugar level measuring device 1 illustrated in Figs. 1 to 4 analyzes a specimen by an electrochemical method using a biosensor 2, and is of a portable type. The blood sugar level measuring device 1 has a housing 3, a connector portion 4, and a disposal mechanism 5.

The housing 3 defines the appearance shape of the blood sugar level measuring device 1, and houses various elements including the connector portion 4 and the disposal mechanism 5. The housing 3 has a casing 30 and a cover 31, and is formed to be hollow.

The casing 30 is formed in a box shape having a top opening 32, and has a stage 33 and an opening 34 provided in an end portion 30A and an engaging section 35 and a stopper portion 36 provided to the inner bottom surface.

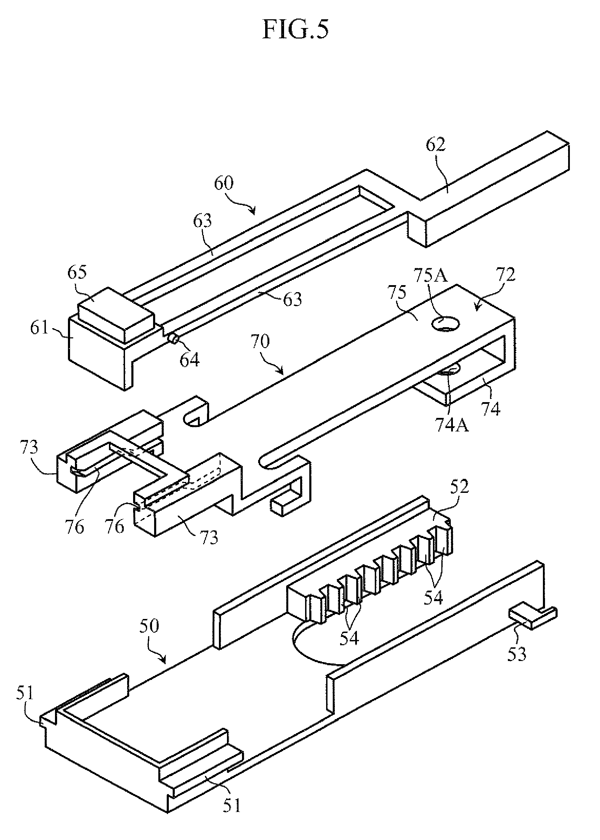

The stage 33 is a portion which holds a guide portion 73 of a fixing body 70 of the later-described disposal mechanism 5 and on which a flange portion 38 of the later-described cover 31 is placed. The opening 34 allows movement of an operating body 50 of the later-described disposal mechanism 5, and exposes the operating body 50. The opening 34 has a notch portion 37 for sliding a guide flange 51 (see Fig. 5) of the operating body 50 of the later-described disposal mechanism 5.

The engaging section 35 is to engage an end portion 11 of a coil spring 10. The stopper portion 36 limits movement of the later-described operating body 50 in the D1 direction and is provided in a position which can interfere with a hook portion 53 of the operating body 50.

The cover 31 covers the top opening 32 of the casing 30, and is formed in its entirety in a plate-like shape. The cover 31 has the flange portion 38 to be brought into close contact with the stage 33 of the casing 30. The flange portion 38 has a notch 38A. The notch 38A defines an insertion opening 39 used for fitting the biosensor 2 into the blood sugar level measuring device 1.

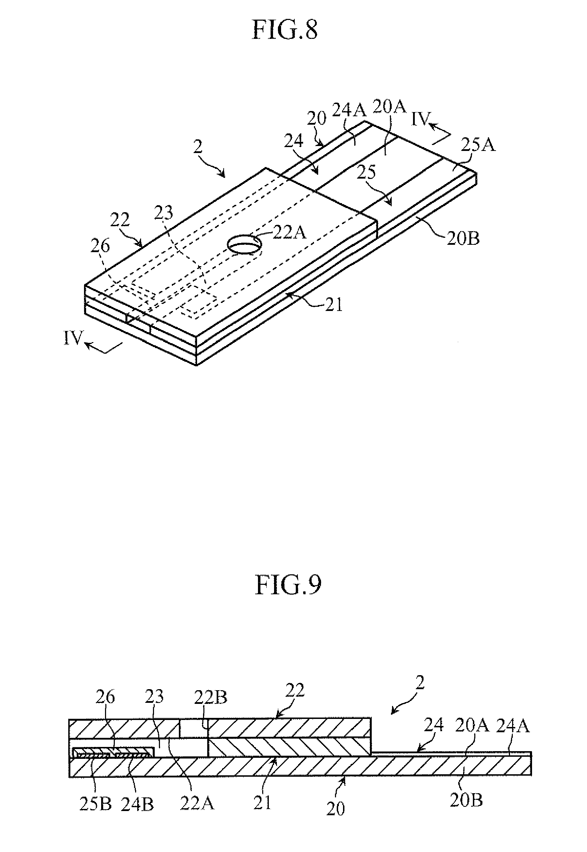

The connector portion 4 fits the biosensor 2 thereinto, and has a pair of terminals 40 (one in the drawing). When the biosensor 2 is fitted into the connector portion 4, the terminals 40 are brought into contact with end portions 24A and 25A (see Fig. 8) of an operating electrode 24 and a counter electrode 25 of the biosensor 2, apply a voltage to the electrodes 24 and 25, and measure its responding current value. The terminal 40 is a plate spring. In addition, when the biosensor 2 is fitted into the connector portion 4, the terminal 40 exerts a pressing force on the biosensor 2 and appropriately holds the biosensor 2 in the connector portion 4.

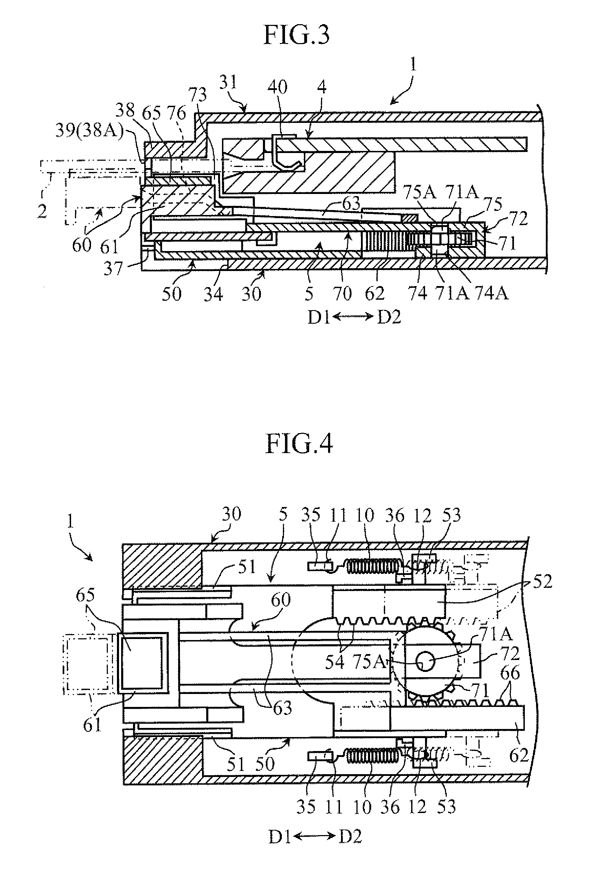

As illustrated in Figs. 3 to 5, the disposal mechanism 5 disposes of the biosensor 2 after completion of blood sugar level measurement from the blood sugar level measuring device 1 and is arranged below the connector portion 4. The disposal mechanism 5 has the operating body 50, a moving body 60, and the fixing body 70.

The operating body 50 is a portion operated to move the moving body 60. The operating body 50 can be reciprocated with respect to the housing 3 in the D1 and D2 directions in a state where a portion of it is exposed from the opening 34 of the housing 3 (casing 30). The operating body 50 has the guide flange 51, a rack portion 52, and the pair of hook portions 53.

The guide flange 51 defines the moving path of the operating body 50, and is guided and moved by the notch portion 37 of the opening 34 in the housing 3 (casing 30).

The rack portion 52 is a portion used for transmitting a power to the moving body 60, and has a plurality of teeth 54. The plurality of teeth 54 are coupled to teeth 66 of a rack portion 62 in the later-described moving body 60 via a gear 71.

Each of the hook portions 53 is to engage an end portion 12 of the coil spring 10. As described above, the end portion 11 of the coil spring 10 engages the engaging section 35 provided in the casing 30. The hook portion 53 can also interfere with the stopper portion 36 of the casing 30. That is, in standby state of the operating body 50, the hook portion 53 interferes with the stopper portion 36 and the coil spring 10 is slightly extended from its natural state. Therefore, the operating body 50 is urged by the coil spring 10 in the D1 direction.

As illustrated in Figs. 3 to 6, the moving body 60 is interlocked with the motion of the operating body 50 so as to be moved in the D1 and D2 directions. The moving body 60 is moved in the D1 direction to move the biosensor 2 fitted into the connector portion 4. The moving body 60 has a pressing portion 61, the rack portion 62, and a pair of plate spring portions 63.

At disposal of the biosensor 2, the pressing portion 61 is brought into contact with a back surface 2A of the biosensor 2 fitted into the connector portion 4. The pressing portion 61 is located immediately below the flange portion 38 of the cover 31 in standby state and is moved to a position completely protruded from the housing 3 at disposal. The pressing portion 61 can be shifted upward and downward at movement in the D1 and D2 directions, and has an engaging pin 64 and a pad portion 65.

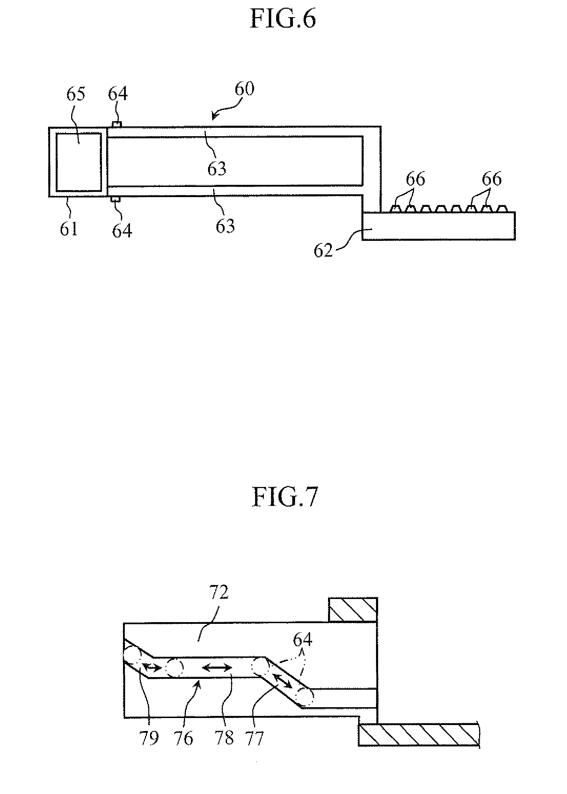

The engaging pin 64 is to engage a guide groove 76 of the later-described fixing body 70, and is moved in the guide groove 76 by movement of the moving body 60 in the D1 and D2 directions (see Fig. 7). The detail will be described later. The engaging pin 64 is moved in the guide groove 76 to shift the pressing portion 61 (pad portion 65) upward and downward.

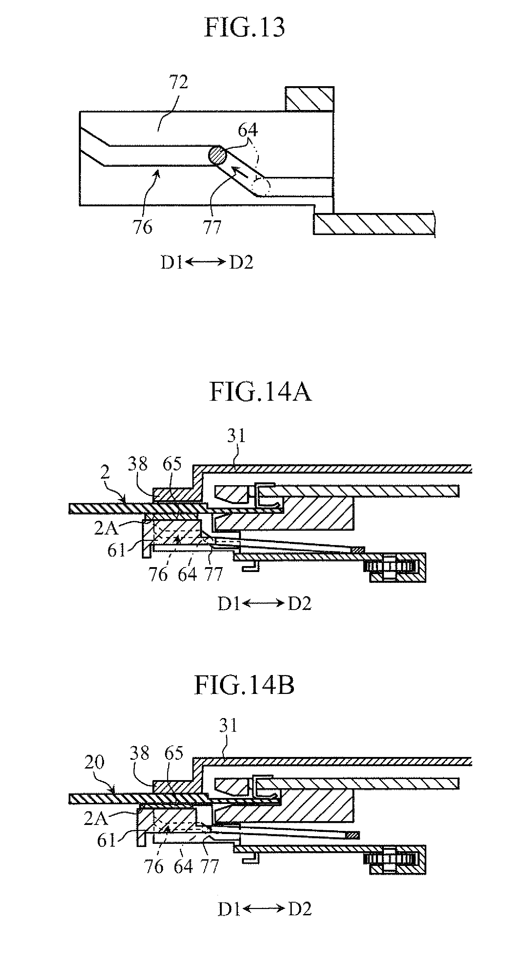

The pad portion 65 is a portion brought into close contact with the back surface 2A of the biosensor 2 at disposal of the biosensor 2. The pad portion 65 in standby state is arranged immediately below the flange portion 38 of the cover 31 so as to form a gap larger than the thickness dimension of the biosensor 2 between the pad portion 65 and the flange portion 38. That is, the pad portion 65 in standby state is arranged in a position which can insert the biosensor 2 into the gap formed between the pad portion 65 and the flange portion 38. Furthermore, the pad portion 65 can be elastically deformed, and is formed of elastomer, foam or the like. That is, when the pad portion 65 is shifted upward, the biosensor 2 is pressed onto the flange portion 38 of the cover 31. so that the pad portion 65 is elastically deformed. The pad portion 65 is thus brought into close contact with the biosensor 2 (see Fig. 14(b)). Therefore, when the pad portion 65 is brought into close contact with the biosensor 2 to move the moving body 60 (pad portion 65) in the D1 direction, the biosensor 2 can be moved together with the moving body 60 (pad portion 65) in the D1 direction by frictional resistance caused between the pad portion 65 and the biosensor 2.

The rack portion 62 is used for inputting a load for moving the moving body 60, and has the plurality of teeth 66. The plurality of teeth 66 are coupled to the plurality of teeth 54 of the operating body 50 via the gear 71.

The pair of plate spring portions 63 connect the pressing portion 61 and the rack portion 62 and have spring properties. The plate spring portions 63 have spring properties, and allow shifting of the pressing portion 61 upward and downward to the rack portion 62.

The fixing body 70 holds the gear 71, and defines the moving path of the pressing portion 61 of the moving body 60. The fixing body 70 has a holder portion 72 and the guide portion 73.

The holder portion 72 rotatably holds the gear 71, and has a pair of plate-like portions 74 and 75 facing each other. The plate-like portions 74 and 75 are extended in a horizontal direction so as to form a gap slightly larger than the thickness dimension of the gear 71 (except for a shaft 71A). The plate-like portions 74 and 75 have through-holes 74Aand 75A into which the shaft 71A of the gear 71 is inserted. The gear 71 has teeth 71B protruded sidewise of the holder portion 72, and is rotatably held in a horizontal direction. The gear 71 engages the teeth 54 and 66 of the rack portions 52 and 62 in the operating body 50 and the moving body 60 in portions where the teeth 71B are protruded from the holder portion 72. When the operating body 50 is moved relatively to the fixing body 70, the moving body 60 is moved in a direction opposite the operating body 50.

As illustrated in Fig. 7, the guide portion 73 shifts the pressing portion 61 upward and downward when the moving body 60 is moved in the D1 and D2 directions, and has the guide groove 76. The guide groove 76 engages the engaging pin 64 of the moving body 60 as described above, and has a low position tilting groove portion 77, a horizontal groove portion 78, and a high position tilting groove portion 79. A series of the groove portions 77, 78, and 79 are connected. The engaging pin 64 is located in the low position tilting groove portion 77 in standby state of the operating body 50 and the moving body 60. When the operating body 50 in standby state is moved in the D2 direction to move the moving body 60 (pressing portion 61) in the D1 direction, the engaging pin 64 is sequentially moved in the low position tilting groove portion 77, the horizontal groove portion 78, and the high position tilting groove portion 79. The engaging pin 64 is shifted upward by movement of the operating body 50 in the D2 direction (or movement of the moving body 60 in the D1 direction). The pressing portion 61 is shifted upward by movement of the operating body 50 in the D2 direction (or movement of the moving body 60 in the D1 direction). In contrast, when the operating body 50 is moved in the D1 direction to move the moving body 60 (pressing portion 61) in the D2 direction, the pressing portion 61 is shifted downward.

As illustrated in Figs. 8 to 10, the biosensor 2 fitted into the blood sugar level measuring device 1 for use is disposal and is formed in its entirety in a planar shape. The biosensor 2 joins a cover 22 to a substrate 20 having a substantially rectangular shape with a spacer 21 interposed therebetween and has a capillary 23 defined by the elements 20 to 22 and extended in a longitudinal direction of the substrate 20.

The spacer 21 defines the distance from a top surface 20A of the substrate 20 to a lower surface 22A of the cover 22, that is, the height dimension of the capillary 23, and is, for example, a double-faced tape. The spacer 21 has a slit 21A for defining the width dimension of the capillary 23.

The cover 22 has an exhaust port 22B for exhausting a gas in the capillary 23 to the outside. The cover 22 is formed of a thermoplastic resin having high wettability, such as vinylon and highly crystallized PVA.

The substrate 20 configures the back surface 2A of the biosensor 2 to be contacted with the pressing portion 61 (pad portion 65) of the disposal mechanism 5 at disposal of the biosensor 2, and is formed of an insulating resin material in a shape larger than the cover 22. The back surface 2A of the substrate 20 (the back surface of the biosensor 2) is preferably subjected to a surface roughening process so as to increase frictional resistance when the pad portion 65 of the pressing portion 61 is brought into contact therewith. The operating electrode 24, the counter electrode 25, and a reagent layer 26 are formed on the top surface 20A of the substrate 20.

The operating electrode 24 and the counter electrode 25 apply a voltage to blood introduced into the capillary 23. The end portions 24A and 25A are exposed from an end portion 20B of the substrate 20 without being covered by the cover 22. When the biosensor 2 is fitted into the blood sugar level measuring device 1, the end portions 24A and 25A are portions brought into contact with the terminal 40 (see Fig. 3) of the connector portion 4. End portions 24B and 25B of the operating electrode 24 and the counter electrode 25 are extended in a shorter direction of the substrate 20. A portion of each of them is located in the capillary 23. The reagent layer 26 is provided so as to cover a series of the end portions 24B and 25B of the operating electrode 24 and the counter electrode 25, and is arranged in the capillary 23. The reagent layer 26 includes such as an electron transfer substance (a chain body such as [Ru(NH₃)₆)]Cl₃ or K₃[Fe(CN)₆] and an oxidoreductase (glucose oxidase(GOD) and glucose dehydrogenase (GDH)), and is formed in a porous solid which is easily dissolved into blood.

The capillary 23 moves blood toward the exhaust port 22B using a capillary phenomenon and holds the introduced blood. When the blood is introduced into the capillary 23, the reagent layer 26 is dissolved to build a liquid phase reaction system including the electron transfer substance, the oxidoreductase, and blood (glucose) in the capillary 23.

The blood sugar level measuring operation using the blood sugar level measuring device 1 and the disposal operation of the biosensor 2 will be described below.

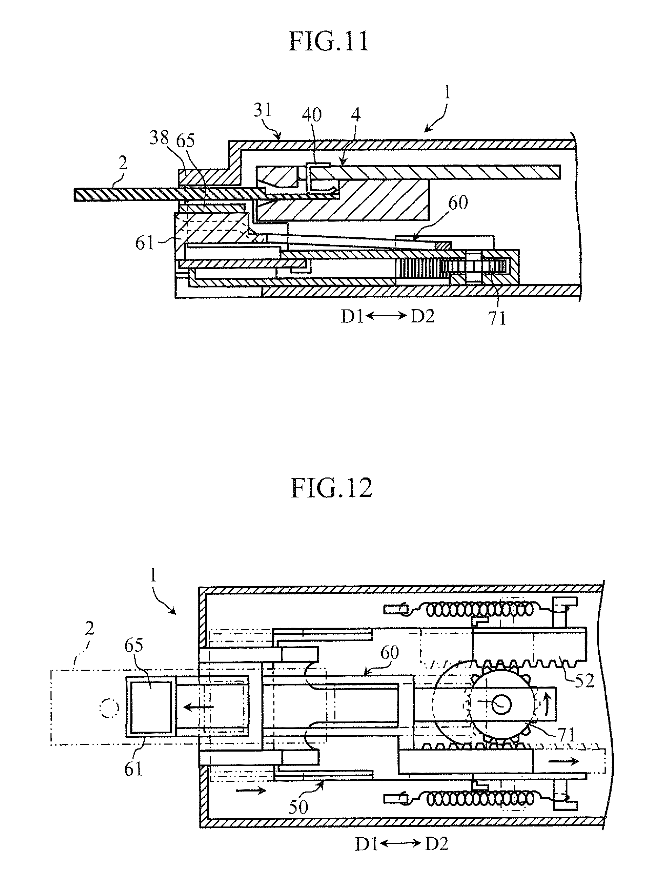

As illustrated in Figs. 11 and 12, when blood sugar level measurement is performed using the blood sugar level measuring device 1, the biosensor 2 is first fitted into the blood sugar level measuring device 1. The biosensor 2 is fitted into the blood sugar level measuring device 1 by inserting the biosensor 2 into the connector portion 4 through the gap between the pad portion 65 of the moving body 60 and the flange portion 38 of the cover 31. At this time, The end portions 24A and 25A (see Fig. 8) of the operating electrode 24 and the counter electrode 25 of the biosensor 2 are brought into contact with the terminal 40 of the connector portion 4. A downward pressing force is thus exerted on the biosensor 2 by the spring properties of the terminal 40. Therefore, the biosensor 2 is appropriately held in the connector portion 4. The terminal 40 of the connector portion 4 is brought into contact with the operating electrode 24 and the counter electrode 25 so as to apply a voltage between the electrodes 24 and 25.

On the other hand, blood is supplied to the biosensor 2 to measure a blood sugar level by the blood sugar level measuring device 1. More specifically, blood is supplied to the biosensor 2 to fill the capillary 23 with the blood. The reagent layer 26 is thus dissolved to build the liquid phase reaction system (see Fig. 9). A voltage is applied between the operating electrode 24 and the counter electrode 25, and is also applied to the liquid phase reaction system. In this way, glucose in the blood is reduced by the oxidoreductase (or electrons are taken out). The electrons are supplied to the end portion 24B of the operating electrode 24 via the electron transfer substance. The amount of the electrons supplied to the operating electrode 24 is measured as a responding current via the terminal 40 of the connector portion 4. The glucose concentration (blood sugar level) is computed by the blood sugar level measuring device 1 based on the responding current.

When the blood sugar level measurement is completed, the biosensor 2 need to be disposed of. In the blood sugar level measuring device 1, a load is exerted on the operating body 50 in standby state in the D2 direction to dispose of the biosensor 2.

When the operating body 50 is moved in the D2 direction, the rack portion 52 is moved in the D2 direction to rotate the gear 71. The moving body 60 is moved in the D2 direction. When the moving body 60 is moved in the D2 direction, the engaging pin 64 of the pressing portion 61 is moved in the guide groove 76 (see Fig. 7).

As illustrated in Fig. 13, the engaging pin 64 is moved in the low position tilting groove portion 77 of the guide groove 76. In this process, the engaging pin 64 is shifted upward. Thus, as illustrated in Fig. 14, the entire of the pressing portion 61 including the pad portion 65 is shifted upward to bring the pad portion 65 into contact with the back surface 2A of the biosensor 2. At this time, the biosensor 2 is pressed onto the flange portion 38 of the case 31 so that the pad portion 65 can be elastically deformed. The pad portion 65 is elastically deformed so as to be appropriately brought into close contact with the back surface 2A of the biosensor 2.

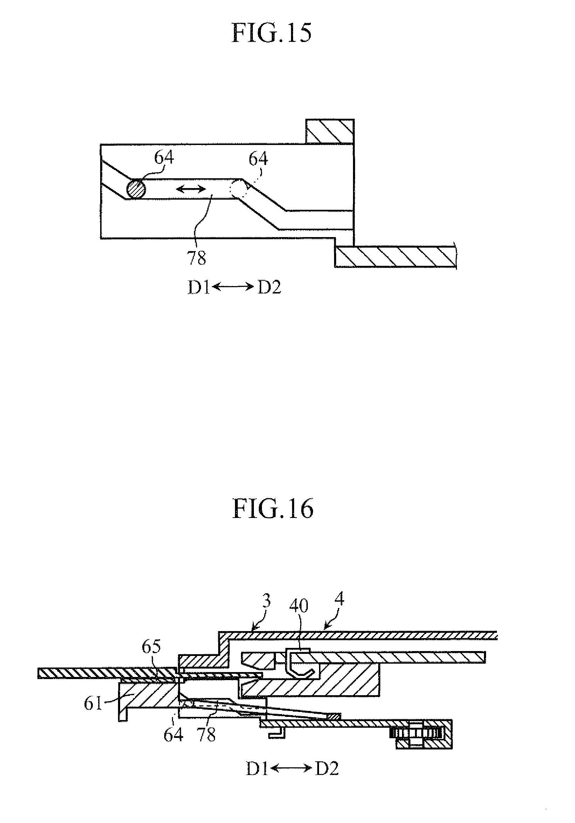

Next, as illustrated in Fig. 15, the engaging pin 64 is moved in the horizontal groove portion 78. In this process, as illustrated in Fig. 16, the entire pressing portion 61 including the engaging pin 64 and the pad portion 65 is moved horizontally in the D1 direction, and finally, the pad portion 65 is completely protruded from the housing 3. The pad portion 65 is brought into close contact with the back surface 2A of the biosensor 2. Accordingly, the biosensor 2 is moved in the D1 direction together with the pad portion 65 by a frictional force caused between the pad portion 65 and the back surface 2A of the biosensor 2. When the back surface 2A of the biosensor 2 is subjected to a surface roughening process, the frictional force caused between the back surface 2A of the biosensor 2 and the pad portion 65 is increased. Accordingly, the biosensor 2 can be moved by the pressing portion 61 more appropriately. As a result, the biosensor 2 is separated from the terminal 40 of the connector portion 4 and thus the pressing force exerted on the biosensor 2 is released. Therefore, the biosensor 2 is free from the connector portion 4.

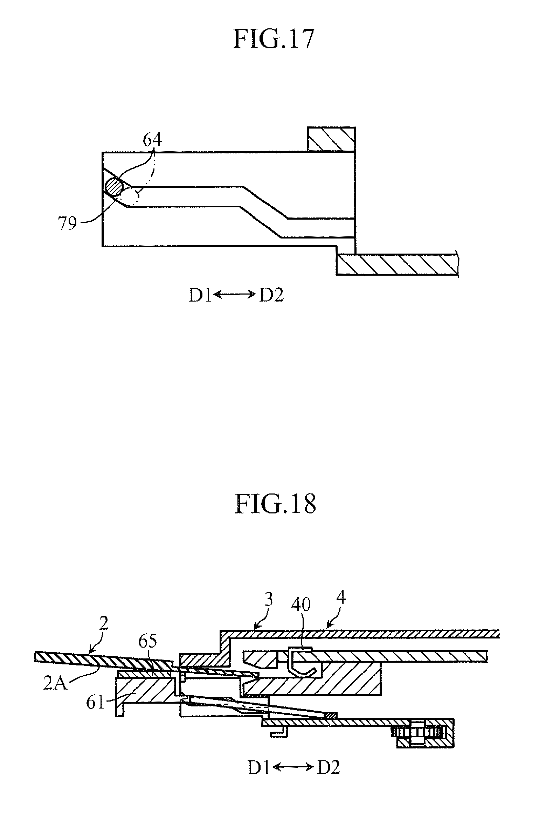

As illustrated in Fig. 17, the engaging pin 64 is moved in the high position tilting groove portion 79. In this process, as illustrated in Fig. 18, the entire pressing portion 61 including the engaging pin 64 and the pad portion 65 is shifted upward outside of the housing 3. The biosensor 2 is shifted upward and interferes with the flange portion 38 of the cover 31 for rotation. The contact state between the back surface 2A of the biosensor 2 and the pad portion 65 is released.

As seen from Figs. 3 and 4, when a load exerted on the operating body 50 in the D2 direction is released, the operating body 50 is moved in the D1 direction by a resilient force of the coil spring 10 so as to be returned to the original position. The hook portion 53 of the operating body 50 interferes with the stopper portion 36 of the housing 3 (casing 30) to limit movement of the moving body 50 in the D1 direction. Therefore, the operating body 50 is appropriately returned to the original position.

On the other hand, when the operating body 50 is moved in the D1 direction, the entire moving body 60 including the pressing portion 61 is moved in the D2 direction. At this time, the pressing portion 61 including the pad portion 65 is shifted downward and the moving body 60 is returned to the original position. Thus, the contact state between the biosensor 2 and the pad portion 65 is completely released. As a result, the biosensor 2 is completely free from the blood sugar level measuring device 1. The pressing portion 61 lowers the blood sugar level measuring device 1 immediately above a trash box. The biosensor 2 is separated from the blood sugar level measuring device 1 so as to be stored in the trash box.

In the blood sugar level measuring device 1, the disposal mechanism 5 is arranged below the connector portion 4 and a load is inputted to the back surface 2A of the biosensor 2 to dispose of the biosensor 2. Unlike the blood sugar level measuring device 9 in the prior art described with reference to Fig. 27, the slit 97 for allowing movement of the interfering portion 95 of the operating knob 92 need not be provided in the connector portion 4. Therefore, the connector portion employed in the blood sugar level measuring device not having the disposal mechanism can be directly diverted without changing the design. Thus, increase in the manufacturing cost due to the design change can be avoided. In addition, when the slit 97 (see Fig. 18) need not be provided in the connector portion 4, the space for the connector portion 4 to provide the slit 97 need not be secured in the connector portion 4. Furthermore, the component of the connector portion 4 is not susceptible to layout limit due to the slit 97. Increase in the size of the connector portion 4 can thus be prevented.

The blood sugar level measuring device is not limited to the previously described configuration and various design changes can be made. For example, the configuration moving the moving body 60 in the D1 direction or the configuration shifting it upward and downward is not limited to the previously described configuration employed in the blood sugar level measuring device 1.

The moving body 60 may be moved in the D1 direction not via the operating body 50 to dispose of the biosensor 2. A load may be inputted from the operating body 50 to the moving body 60 using rotation of a cam or a rod as well as the gear 71. The engaging pin is provided in the fixing body 70 and the guide groove is provided in the moving body 60,so that the pressing portion 61 can also be shifted upward and downward.

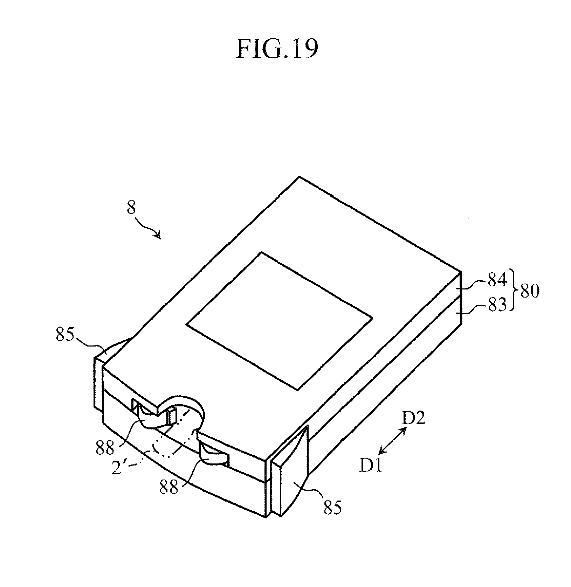

A second embodiment of the present invention will be described below with reference to Figs. 19 to 24.

A blood sugar level measuring device 8 illustrated in Figs. 19 to 21 analyzes a specimen by an electrochemical method using a biosensor 2', and is of a portable type. The blood sugar level measuring device 8 has a housing 80, a connector portion 81, and a disposal mechanism 82.

The housing 80 defines the appearance shape of the blood sugar level measuring device 8, and houses various elements including the connector portion 81 and the disposal mechanism 82. The housing 80 has a casing 83 and a cover 84, and is formed to be hollow.

The casing 83 rotatably supports an operating body 85 and a rotor 88 of the later-described disposal mechanism 82. The casing 83 is formed in a box shape having a top opening, and has a guide 83A, an engaging portion 83B, and a guide groove 83C provided to the inner bottom surface.

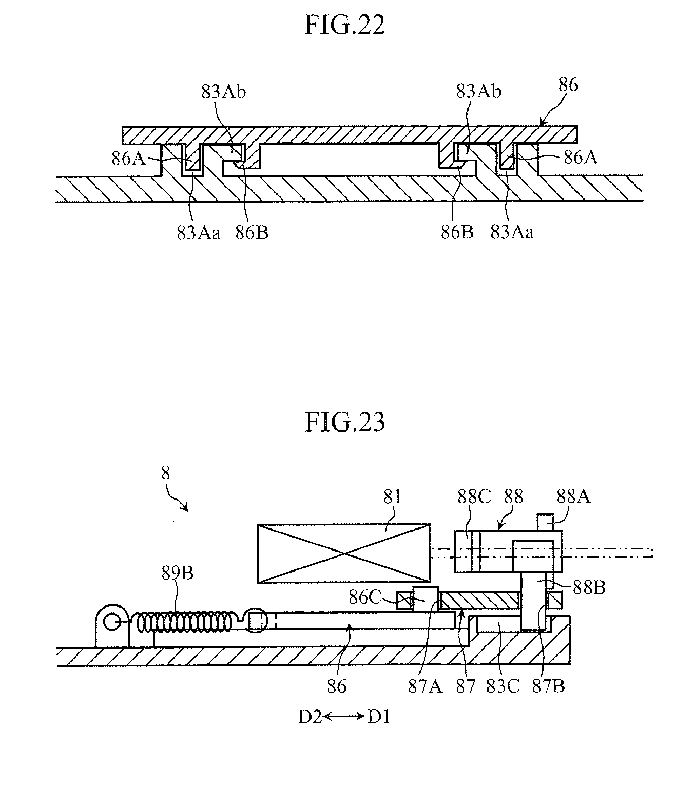

As illustrated in Fig. 22, the guide 83A defines the moving path of a driving base 86 of the later-described disposal mechanism 82. The guide 83A has a groove portion 83Aa and a flange portion 83Ab. The groove portion 83Aa is a portion for moving a rail 86A of the later-described driving base 86. The flange portion 83Ab is a portion for engaging a hook 86B of the later-described driving base 86.

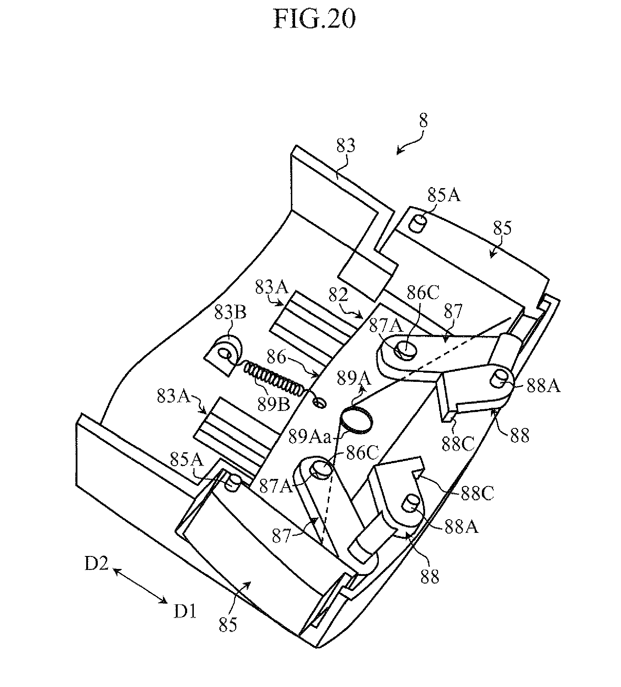

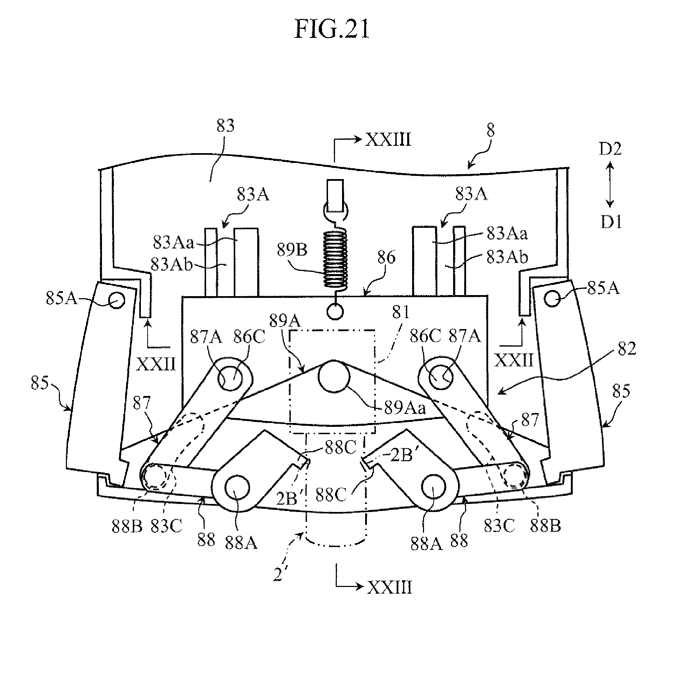

As illustrated in Figs. 20 and 21, the engaging portion 83B is to engage the end portion of a coil spring 89B of the later-described disposal mechanism 82.

The guide groove 83C defines the moving path of a link shaft 88B of the rotor 88 of the later-described disposal mechanism 82.

As illustrated in Fig. 19, the cover 84 covers the top opening of the casing 83, and rotatably supports the operating body 85 and the rotor 88 of the later-described disposal mechanism 82 together with the casing 83.

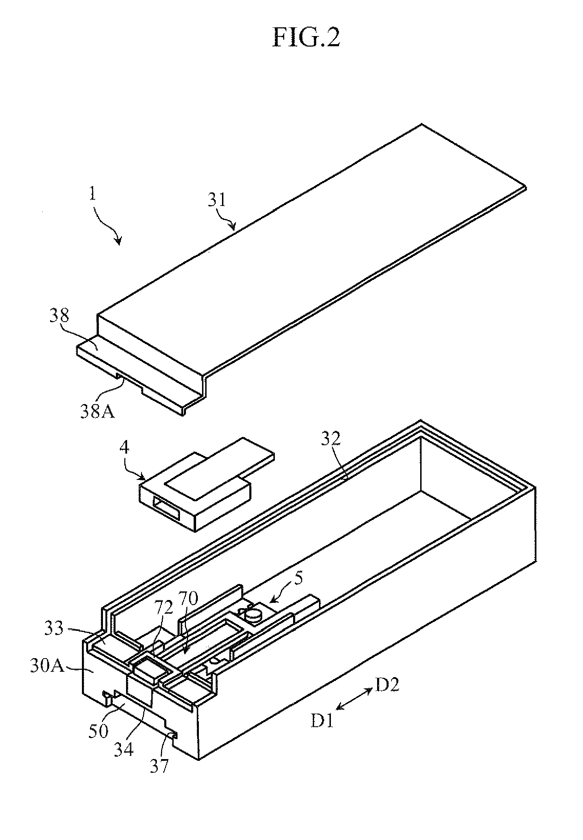

As illustrated in Figs. 21 and 23, the connector portion 81 fits the biosensor 2' thereinto, and has the same function and configuration as those of the connector portion 4 (see Fig. 2 and the like) of the blood sugar level measuring device 1 described above.

As illustrated in Figs. 20, 21 and 23, the disposal mechanism 82 disposes of the biosensor 2' after completion of blood sugar level measurement from the blood sugar level measuring device 8, and is arranged in its entirety below the connector portion 81. The disposal mechanism 82 has the operating body 85, the driving base 86, a link arm 87, and the rotor 88.

The operating body 85 is a portion operated to rotate the rotor 88 and a portion thereof is exposed from the housing 80. The operating body 85 has an engaging pin 85A, and is fixed to the housing 80 (casing 83 and cover 84) so as to be rotatable about the engaging pin 85A.

The driving base 86 transmits a load inputted from the operating body 85 to the rotor 88, and can be reciprocated in the D1 and D2 directions in the drawing. As illustrated in Fig. 22, the driving base 86 has the rail 86A and the hook 86B. The rail 86A is a portion moving the groove portion 83Aa of the guide 83A. The hook 88B is a portion engaging the flange portion 83Ab of the guide 83A. The moving path of the driving base 86 is restricted by the guide 83A in the D1 and D2 directions in the drawing. The hook 88B engages the flange portion 83Ab. Thus, the separation of the driving base 86 from the casing 83 can be prevented.

As illustrated in Figs. 20 and 23, the driving base 86 is coupled to the operating body 85 via a torsion spring 89A, and is coupled to the engaging portion 83B of the casing 83 via the coil spring 89B.

The torsion spring 89A is coupled to the operating body 85 in a portion away from an engaging pin 84A of the operating body 85, and is fixed to the driving base 86 in a ring-like portion 89Aa. When no load is inputted to the operating body 85, the torsion spring 89A exerts an outward force on the operating body 85. On the other hand, when an inward load is inputted to the operating body 85, the end portion of the torsion spring 89A is moved inward and the ring-like portion 89Aa of the torsion spring 89A is moved in the D2 direction in the drawing. Accordingly, the driving base 86 is moved in the D2 direction when the inward load is inputted to the operating body 85.

The coil spring 89B functions as a return spring for moving the driving base 86 in the D1 direction. When an inward load is inputted to the operating body 85 to move the driving base 86 in the D2 direction, the coil spring 89B is shrunk. On the other hand, when the load inputted to the operating body 85 is released, the coil spring 89B moves the driving base 86 in the D1 direction by a resilient force.

The link arm 87 rotates the rotor 88 according to the motion of the driving base 86 in the D1 and D2 directions. The link arm 87 is rotatably coupled to a protrusion 86C of the driving base 86 through a through-hole 87A.

The rotor 88 exerts a load on the side surface (a concave portion 2B') of the biosensor 2' to move the biosensor 2' in the D1 direction, and has a rotating shaft 88A, the link shaft 88B, and a contacting portion 88C.

The rotating shaft 88A is the center of the rotor 88, and is rotatably supported by the housing 80 (casing 83 and cover 84).

The link shaft 88B links the rotor 88 and the link arm 87, and is inserted into a through-hole 87B of the link arm 87. An end portion 88Ba of the link shaft 88B is inserted into the guide groove 83C of the casing 83. Accordingly, when the link arm 87 is moved together with the driving base, the link shaft 88B is moved along the guide groove 83C. By such movement of the link shaft 88B, the rotor 88 is rotated about the rotating shaft 88A.

The contacting portion 88C is a portion brought into contact with the side surface (concave portion 2B') of the biosensor 2' when the biosensor 2' fitted into the connector portion 81 is moved in the D1 direction (or is disposed of). The contacting portion 88C is arranged in a position corresponding to the concave portion 2B' of the biosensor 2' when the biosensor 2' is fitted into the connector portion 81 and no load is inputted to the operating body 85.

As illustrated in Fig. 21, the biosensor 2' fitted into the blood sugar level measuring device 9 for use is disposal and is formed in its entirety in a planar shape. The biosensor 2' has basically the same configuration as that of the previously described biosensor 2 (see Figs. 8 to 10). However, the biosensor 2' has the pair of concave portions 2B' provided in a side surface 2B. The concave portion 2B' engages the contacting portion 88C of the rotor 88 when the biosensor 2' is disposed of by the disposal mechanism 82.

The blood sugar level measuring operation of the blood sugar level measuring device 9 described above is basically the same as that of the previously described blood sugar level measuring device 1 (see Figs. 1 to 3 and the like). That is, when a blood sugar level is measured, the biosensor 2' is first inserted into the blood sugar level measuring device 9 to fit the biosensor 2' into the connector portion 81. Blood is next supplied to the biosensor 2'. In this way, the blood sugar level measuring device 9 measures a blood sugar level based on a responding current when a voltage is applied by the connector portion 81.

On the other hand, the disposal operation of the blood sugar level measuring device 9 is performed by exerting an inward load inputted to the operating body 85.

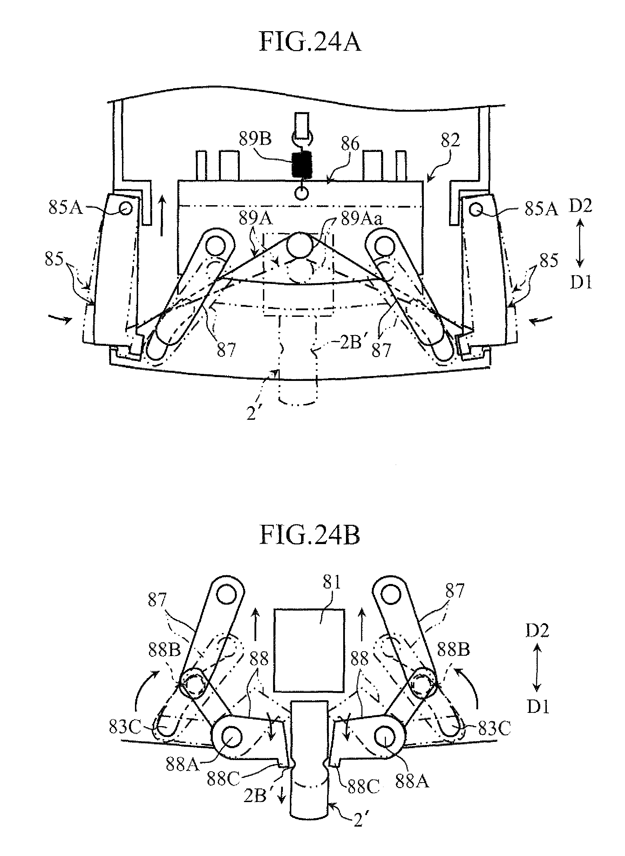

As illustrated in Fig. 24A, when an inward load is exerted on the operating body 85, the operating body 85 is rotated about the engaging pin 85A. At this time, the end of the torsion spring 89A is moved inward and the ring-like portion 89Aa of the torsion spring 89A is moved in the D2 direction. The ring-like portion 89Aa of the torsion spring 89A is fixed to the engaging portion 83B of the driving base 86. Accordingly, movement of the ring-like portion 89Aa in the D2 direction moves the driving base 86 in the D2 direction. The driving base 86 is coupled to the link arm 87 and the coil spring 89B. Accordingly, movement of the driving base 86 in the D2 direction moves the entire link arm 87 in the D2 direction to shrink the coil spring 89B.

As illustrated in Fig. 24B, the link shaft 88B of the rotor 88 is inserted into the through-hole 87A of the link arm 87. Accordingly, movement of the link arm 87 moves the link shaft 88B in the guide groove 83C. This swings the link shaft 88B so that the rotor 88 is rotated about the rotating shaft 88A. The contacting portion 88C of the rotor 88 is rotated along the D1 direction.

In this case, the contacting portion 88C of the rotor 88 is arranged in a position corresponding to the concave portion 2B' of the biosensor 2'. When the contacting portion 88C is rotated along the D1 direction, the contacting portion 88C engages the concave portion 2B' of the biosensor 2' to exert a load onto the biosensor 2' in the D1 direction via the concave portion 2B'. Therefore, the biosensor 2' is detached from the connector portion 81.

As predicted from Fig. 24A, the inward load acting on the operating body 85 is released. The operating body 85 is rotated outward by a resilient force of the torsion spring 89A and is then returned to the original position. The driving base 86 is moved in the D1 direction and is then returned to the original position. The resilient force of the coil spring 89B is exerted on the driving base 86. The driving base 86 can be moved in the D1 direction appropriately and smoothly. As predicted from Fig. 24B, such rotation of the operating body 85 and movement of the driving base 86 return the link arm 87 and the rotor 88 to the original position.

In the blood sugar level measuring device 8, the disposal mechanism 82 is arranged below the connector portion 81 and a load is inputted to the side surface (concave portion 2B') of the biosensor 2' to dispose of the biosensor 2. Unlike the blood sugar level measuring device 9 in the prior art described with reference to Fig. 27, the slit 97 for allowing movement of the interfering portion 95 of the operating knob 92 need not be provided in the connector portion 4. Therefore, the connector portion employed in the blood sugar level measuring device not having the disposal mechanism can be directly diverted without changing the design. Thus, increase in the manufacturing cost due to the design change can be avoided. In addition, when the slit 97 (see Fig. 18) need not be provided in the connector portion 4, the space for the connector portion 4 to provide the slit 97 need not be secured in the connector portion 4. Furthermore, the component of the connector portion 4 is not susceptible to layout limit due to the slit 97. Increase in the size of the connector portion 4 can thus be prevented.

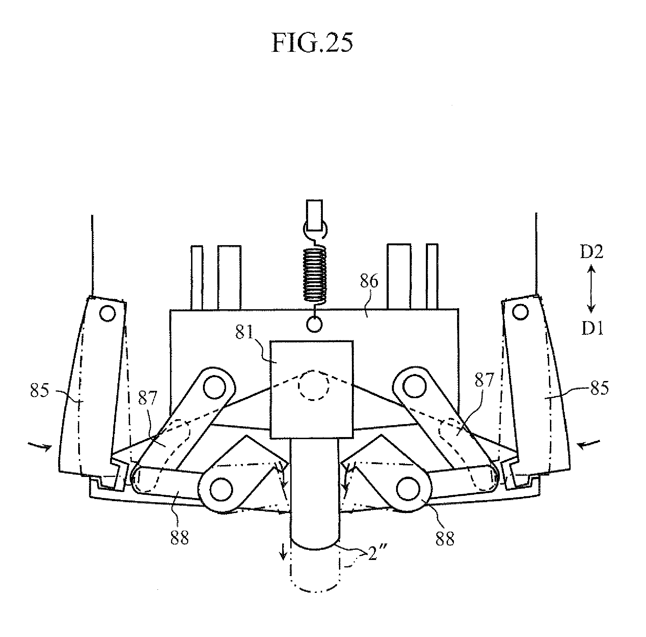

The shape of the concave portion 2B' of the biosensor 2' is not limited to the illustration and may be of other shape. In place of the concave portion 2B', an engaging portion may be formed as a convex portion. As illustrated in Fig. 25, the blood sugar level measuring device 8 is not limited to the biosensor 2' having a side surface formed with the concave portion 2B', and is applicable to a biosensor 2" whose side surface is flat.

The analyzing device according to the present invention may input a load to the analyzing instrument in a direction different from the disposal direction at disposal of the analyzing instrument such as the biosensor, and is applicable to disposal of the analyzing instrument not of the planar shape.

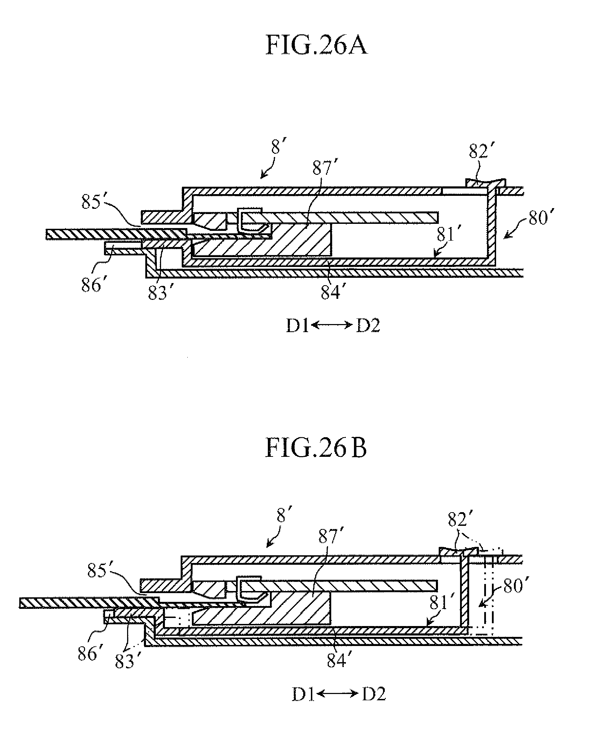

A third embodiment of the present invention will be described below with reference to Fig. 26.

An analyzing device 8' illustrated in Figs. 26A and 26B has a disposal mechanism. A disposal mechanism 80' has a moving body 81'. The moving body 81' is moved at disposal of the biosensor 2, and has an operating body 82', a contacting portion 83', and a coupling arm 84'.

The operating body 82' is a portion operated to move the moving body 81'. The contacting portion 83' is a portion brought into contact with the back surface of the biosensor 2 and moving the biosensor 8' by frictional resistance between the contacting portion 83' and the biosensor 2. The contacting portion 83' is moved in the D1 and D2 directions along a guide 86' provided below an insertion opening 85', and is brought into close contact with the back surface of the biosensor 2 when the biosensor 2 is fitted into a connector portion 87'. The coupling arm 84' couples the operating portion 82' and the contacting portion 83', and transmits a load inputted from the operating body 82' to the contacting portion 83' to move the contacting portion 83'.

In the analyzing device 8', the biosensor 2 is fitted into the connector portion 87' to bring the back surface of the biosensor 2 into close contact with the contacting portion 83'. When the biosensor 8' is disposed of, the operating portion 82' is moved in the D1 direction to move the contacting portion 82' in the D1 direction. The contacting portion 8' is brought into close contact with the biosensor 2. A force is thus exerted on the biosensor 2 in the D1 direction to move the biosensor 2 in the D1 direction. Therefore, the biosensor 2 is separated from the connector portion 87' so that the biosensor 2 can be disposed of.

In the previous embodiments, the analyzing device configured to measure the blood sugar level by the electrochemical method is described. The present invention is applicable to an analyzing device using a specimen other than blood, an analyzing device configured to measure an ingredient other than glucose, or an analyzing device configured to analyze a specimen by an optical method.

When the previously described disposal mechanism 5 is employed in the analyzing device using the optical method, unlike the blood sugar level measuring device 9 described with reference to Fig. 18, the slit 99 for allowing movement of the interfering portion 95 of the operating knob 92 need not be provided in the housing 98. Therefore, an external light cannot be incident into the device through the slit 98 of the housing 98. The photometry result of the photometer mechanism reduces the possibility of the influence of the external light, thereby performing appropriate photometry. Furthermore, means for limiting incidence of the external light through the slit 98 need not be provided. Increase in the manufacturing cost and size of the analyzing device can thus be prevented.

## Claims

1. An analyzing device which fits an analyzing instrument thereinto for use and has a disposal mechanism for disposing of the analyzing instrument after completion of analysis,
wherein the disposal mechanism inputs a load in a direction different from a disposal direction of the analyzing instrument to dispose of the analyzing instrument.

2. An analyzing device comprising:
a connector portion which fits thereinto an analyzing instrument which can analyze a specimen by an electrochemical method and has a terminal brought into contact with a terminal of the analyzing instrument; and
a disposal mechanism for disposing of the analyzing instrument after completion of analysis,
wherein the disposal mechanism is arranged below the connector portion.

3. The analyzing device having the disposal mechanism according to claim 2, wherein the disposal mechanism inputs a load in a direction different from a disposal direction of the analyzing instrument to dispose of the analyzing instrument.

4. The analyzing device having the disposal mechanism according to claim 1 or 3,
wherein the disposal mechanism has a moving body which has a contacting portion brought into close contact with the analyzing instrument and can be reciprocated between a standby position and a disposal position which can dispose of the analyzing instrument,
wherein when a load is inputted by the contacting portion and the moving body is moved from the standby position to the disposal position, the analyzing instrument is moved together with the moving body.

5. The analyzing device having the disposal mechanism according to claim 4, wherein the analyzing instrument contacted with the contacting portion is moved together with the moving body due to frictional resistance between the analyzing instrument and the contacting portion.

6. The analyzing device having the disposal mechanism according to claim 5, wherein the contacting portion is formed of an elastic member.

7. The analyzing device having the disposal mechanism according to claim 4, wherein when the moving body is in the standby position, the contacting portion is not brought into contact with the analyzing instrument, and when the moving body is moved from the standby position to the disposal position, the contacting portion is brought into contact with the analyzing instrument.

8. The analyzing device having the disposal mechanism according to claim 7, wherein the contacting portion is shifted upward while the moving body is moved from the standby position to the disposal position.

9. The analyzing device having the disposal mechanism according to claim 8, further comprising an engaging portion which engages the moving body and can be moved relatively to the moving body,
wherein one of the moving body and the engaging portion has a guide groove and the other has a convex portion which engages the guide groove,
wherein when the moving body is reciprocated relatively to the housing, the convex portion is moved in the guide groove to shift the contacting portion upward and downward.

10. The analyzing device having the disposal mechanism according to claim 8, further comprising the pressing portion which sandwiches the analyzing instrument between the pressing portion and the contacting portion when the contacting portion is shifted upward.

11. The analyzing device having the disposal mechanism according to claim 4, further comprising:
an operating body which can be reciprocated relatively to the housing; and
a link member for converting the reciprocating motion of the operating body to the reciprocating motion of the moving body.

12. The analyzing device having the disposal mechanism according to claim 11,
wherein the operating body and the moving body each have a rack portion having a plurality of teeth,
wherein the link member is a gear which engages the plurality of teeth of the rack portion of the operating body or the moving body, and
wherein when the operating body is moved, the moving body is moved in a direction opposite the operating body.

13. The analyzing device having the disposal mechanism according to claim 12, wherein the gear is rotated in a horizontal direction.

14. The analyzing device having the disposal mechanism according to claim 11, wherein the operating body is urged in a direction from the standby position to the disposal position.

15. The analyzing device having the disposal mechanism according to claim 14, further comprising a stopper portion for locating the operating body in the standby position.

16. The analyzing device having the disposal mechanism according to claim 1 or 3, wherein the disposal mechanism inputs a load to the side surface of the analyzing instrument to dispose of the analyzing instrument.

17. The analyzing device having the disposal mechanism according to claim 16,
wherein the disposal mechanism has a rotor which has a contacting portion brought into close contact with the analyzing instrument and is rotatable,
wherein the analyzing instrument is moved by inputting a load by the contacting portion and rotating the rotor.

18. The analyzing device having the disposal mechanism according to claim 17, wherein the disposal mechanism further has an operating body operated to rotate the rotor.

19. The analyzing device having the disposal mechanism according to claim 18, wherein the disposal mechanism further has a link member for transmitting a load inputted to the operating body as the rotational force of the rotor.

20. The analyzing device having the disposal mechanism according to claim 17, wherein the analyzing instrument has an engaging portion for engaging the contacting portion.

21. An analyzing device which fits an analyzing instrument thereinto for use and has a disposal mechanism which disposes of the analyzing instrument after completion of analysis,
wherein the disposal mechanism has a moving body which has a contacting portion brought into close contact with the analyzing instrument and can be reciprocated between a standby position and a disposal position which can dispose of the analyzing instrument, wherein when a load is inputted by the contacting portion and the moving body is moved from the standby position to the disposal position, the analyzing instrument is moved together with the moving body.
